# EUROPEAN PATENT SPECIFICATION

(11) **EP 2 863 761 B1**
(45) Date of publication and mention of the grant of the patent: **27.06.2018**
(21) Application number: 13725886.9
(22) Date of filing: 22.05.2013
(51) Int. Cl.: A23L 33/135, A23L 33/18, A61K 35/74, A61P 1/00, A61K 38/01

(54) **DIETARY MANAGEMENT OF CELIAC DISEASE AND FOOD ALLERGY**
DIETÄTISCHE BEHANDLUNG VON ZÖLIAKIE UND LEBENSMITTELALLERGIE
PRISE EN CHARGE DIÉTÉTIQUE DE LA MALADIE C LIAQUE ET DES ALLERGIES ALIMENTAIRES

(30) Priority: 20.06.2012 US 201213528818
(43) Date of publication of application: 29.04.2015
(73) Proprietor: MJN U.S. Holdings LLC, Chicago, Illinois 60606 (US)
(72) Inventor: TANBONLIONG, Gretchen, Honolulu, Hawaii 96818-4630 (US)
(74) Representative: Gill-Carey, Michael
(86) International application number: PCT/US2013/042133
(87) International publication number: WO 2013/191845

(56) References cited:
- EP-A1- 0 904 784
- WO-A1-98/55131
- WO-A1-2007/108763
- WO-A1-2010/130701
- WO-A2-2005/027953
- US-A1- 2006 233 915
- US-A1- 2011 070 334

## Description

### TECHNICAL FIELD

The present disclosure relates to methods and compositions for the dietary management of celiac disease and of food allergy in a human subject via enteral administration of at least one hydrolyzed protein component and a probiotic component comprising *Lactobacillus rhamnosus* GG. The disclosure further relates to nutritional compositions, such as infant formulas or nutritional supplements, comprising the probiotic *Lactobacillus rhamnosus* GG together with at least one extensively hydrolyzed protein component for managing, ameliorating or alleviating the symptoms of celiac disease and/or milk protein allergy and for promoting healthy intestinal growth and development in a subject.

### BACKGROUND ART

Celiac disease ("CD") is an autoimmune-mediated intestinal disorder that affects genetically predisposed individuals when they ingest prolamins present in wheat (gliadin), rye (secalin), barley (hordein) or any crossbred varieties thereof. (Rizello et al., Appl Environ Microbiol. 2007 Jul;73(14):4499-4507). In celiac disease patients, ingestion of a prolamin often induces severe intestinal symptoms and inflicts small-bowel mucosal damage because the patients lack an oral tolerance to wheat gluten and/or to related prolamins. For example, in most CD patients, ingestion of gluten leads to activation of both an adaptive immune response dominated by Th1 pro-inflammatory cytokines and an innate immune response mediated by interleukin-15 (IL-15). (Nadal et al., J Med Microbiol. 2007 Dec; 56(Pt 12):1669-74). In its active phase, celiac disease may also alter epithelial permeability and tight junction integrity, thereby allowing antigens to access the submucosa.

Celiac disease is one of the most common food-related disorders in western countries, and an estimated 1-3% of European and American populations are affected. The disease can manifest at any age and may present a variety of clinical features. Generally, though, it presents in early childhood via small intestinal villous atrophy and signs of malabsorption, which may result in failure to thrive and/or severe malnutrition in children and/or infants.

The early onset of CD has been attributed to several genetic and environmental causes. Yet, at present the only effective treatment for the disease is a strict life-long gluten-free diet. (Lindfors et al., Clin Exp Immunol. 2008 Jun; 152(3):552-558).

The Codex Alimentarius Commission of the World Health Organization and the Food and Agriculture Organization of the United Nations distinguish gluten-free foods as those consisting of ingredients with a gluten level of <20 ppm or those that have been rendered gluten free with a gluten level of <200 ppm. (Rizello et al., Appl. Environ. Microbiol. 2007 Jul;73(14):4499-4507). To achieve "gluten-free" status, gluten in foods can be detoxified after ingestion in the gastrointestinal tract, or it may be hydrolyzed prior to ingestion during food processing. (Lindfors et al.).

Previous studies have suggested that use of probiotic bacteria in sourdough fermentation can induce the hydrolysis of gluten during food processing and thus be beneficial in the process of providing gluten-free foods for celiac disease patients. (Lindfors et al.). Indeed, the manufacture of wheat and rye breads or pasta with flours made tolerable to CD patients by using selected sourdough fermentation processes may decrease the toxicity of certain epitopes, but the concentration of gluten in these foods generally remains above 6,000 ppm. (Rizello et al.).

There have also been recent efforts to develop wheat that is free of celiac disease epitopes. *Id.* Yet, development of wheat varieties that are free of toxic polypeptide sequences is considered unlikely because of the high degree of sequence homology existing among members of the cereal protein family because cereals, like wheat, are hexaploid. *Id.* Accordingly, new treatment options are warranted. WO 2007/108763 describes a method of promoting healthy intestinal development in an infant suffering from celiac disease with Lactobacillus rhamnosus, EP 0 904 784 describes a method of promoting healthy intestinal development in an infant optionally including celiac disease, comprising the step of: administering to the infant optionally probiotic Lactobacillus rhamnosus GG and protein hydrolysates, all to be selected from long lists.

Furthermore, though CD patients adhere to a strict gluten-free diet, it is possible that CD patients may still develop additional food allergies. For example, dairy consumption is currently permitted as part of the life-long gluten-free diet regimen. Yet, as the bowel of a CD patient becomes progressively more damaged due to the effects of the disease, a degree of lactose intolerance may develop in some patients. Moreover, some CD patients develop food allergy, such as cow's milk allergy.

Food allergy is an immunologically mediated clinical syndrome that may develop after the ingestion of a dietary product. The adverse reaction that accompanies a food allergy is often an immediate immunoglobulin-E (IgE) mediated reaction, otherwise known as a food protein allergy. (Host, A., et al., Dietary Products Used in Infants for Treatment and Prevention of Food Allergy, Arch. Dis. Child 81:80-84 (1999)). Symptoms of food protein allergy include angioedema, urticaria, exzema, asthma, rhinitis, conjunctivitis, vomiting, and/or anaphylaxis.

Cow's milk allergy is the most common food protein allergy in young children and occurs in about 2% to 3% of all infants. (Sampson, H. A., Food Allergy. Part 1: Immunopathogenesis and Clinical Disorders, J Allergy Clin Immunol. 103:717-728 (1999)). The cow's milk protein used in most infant formulas is considered a foreign protein. When infants are exposed to non-human milk, they can develop antibodies to the foreign protein. Research has shown that the important food allergens found in both milk and soybean formulas are stable to digestion in the stomach for as long as 60 minutes (as compared to human milk protein which is digested in the stomach within 15 minutes). The foreign proteins then pass through the stomach and reach the intestines intact, where they gain access and can cause sensitization. The infant's immune system then "attacks" the foreign proteins, resulting in symptoms of an allergic reaction.

One possible explanation for the prevalence of protein allergies among infants is that intact cow's milk protein is the earliest and most common food allergen to which infants are exposed.

Accordingly, there is a need for nutritional compositions, such as infant formulas, that are well-tolerated by patients suffering from celiac disease and/or from food allergy, such as cow's milk allergy, that minimize inclusion of immune-triggering epitopes.

### DISCLOSURE OF THE INVENTION

Briefly, therefore, the present disclosure is directed to methods and compositions for dietary management of celiac disease and/or food allergy, such as cow's milk protein allergy, via administration of a nutritional composition that includes an effective amount of *Lactobacillus rhamnosus* GG together with a hydrolyzed protein. The invention is best described by the independent claims.

The disclosure is directed to a nutritional composition comprising a protein component comprising extensively hydrolyzed protein and at least one probiotic, wherein the at least one prohiotic comprises Lactobacillus rhamnosus GG, for use in a method of promoting healthy intestinal development in an infant suffering from celiac disease. Further, the disclosure is directed to a nutritional composition comprising a protein component comprising hydrolyzed protein and at least one probiotic for use in alleviating the symptoms of celiac disease in an infant suffering from celiac disease wherein the nutritional composition further comprises a lipid component and the at least one probiotic comprises Lactobacillus rhamnosus GG.

### BEST MODE FOR CARRYING OUT THE INVENTION

Reference now will be made in detail to the embodiments of the present disclosure, one or more examples of which are set forth below. Each example is provided by way of explanation, not as a limitation. In fact, it will be apparent to those skilled in the art that various modifications and variations can be made to the present disclosure. For instance, features illustrated or described as part of one embodiment, can be used on another embodiment to yield a still further embodiment. Thus, it is intended that the present disclosure covers such modifications and variations as come within the scope of the appended claims and their equivalents.

### Definitions

Hereinafter, "enteral administration" includes, but is not limited to, feeding via nasogastric tube, orogastric feeding, intragastric feeding, transpyloric administration, oral administration and/or rectal administration. "Enteral administration" may also include any other method known in the art for introducing a nutritional composition into the digestive tract.

The term "nutritional composition" includes nutritional supplements, infant formulas, child's milk formulas, growing-up milks, human milk fortifiers, infant formula fortifiers, and the like, but is not limited to the same. The nutritional compositions of the present disclosure may be suitable for enteral administration.

The term "infant" means a human subject ranging in age from birth to not more than about one year and includes infants from 0 to about 12 months corrected age. The phrase "corrected age" means an infant's chronological age minus the amount of time that the infant was born premature. Therefore, the corrected age is the age of the infant if it had been carried to full term. The term infant includes low birth weight infants, very low birth weight infants, extremely low birth weight infants and preterm or premature infants.

The term "child" means a human between the ages of about 1 and 12 years of age. In certain embodiments, a child is between the ages of about 1 and 6 years. In other embodiments, a child is between the ages of about 7 and 12 years.

The term "degree of hydrolysis" refers to the extent to which peptide bonds are broken by a hydrolysis method.

The term "partially hydrolyzed" means having a degree of hydrolysis which is greater than 0% but less than about 50%.

The term "extensively hydrolyzed" means having a degree of hydrolysis which is greater than or equal to about 50%.

The term "protein-free" means containing no measurable amount of protein, as measured by standard protein detection methods such as sodium dodecyl (lauryl) sulfate-polyacrylamide gel electrophoresis (SDS-PAGE) or size exclusion chromatography.

The term "probiotic" means a live, active or viable microorganism that exerts beneficial effects on the health of the host.

The term "inactivated probiotic" or "inactivated LGG" means a probiotic wherein the metabolic activity or reproductive ability of the referenced probiotic or LGG organism has been reduced or destroyed. The "inactivated probiotic" or "inactivated LGG" does, however, still retain, at the cellular level, at least a portion its biological glycol-protein and DNA/RNA structure. As used herein, the term "inactivated" is synonymous with "non-viable".

All percentages, parts and ratios as used herein are by weight of the total composition, unless otherwise specified.

The nutritional composition of the present disclosure may also be substantially free of any optional or selected ingredients described herein, provided that the remaining nutritional composition still contains all of the required ingredients or features described herein. In this context, and unless otherwise specified, the term "substantially free" means that the selected composition contains less than a functional amount of the optional ingredient, typically less than 0.1% by weight, and also, including zero percent by weight of such optional or selected ingredient.

All references to singular characteristics or limitations of the present disclosure shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

The methods and compositions of the present disclosure, including components thereof, can comprise, consist of, or consist essentially of the essential elements and limitations of the embodiments of the disclosure described herein, as well as any additional or optional ingredients, components or limitations described herein or otherwise useful in nutritional compositions.

As used herein, the term "about" should be construed to refer to both of the numbers specified in any range. Any reference to a range should be considered as providing support for any subset within that range.

### Embodiments

The present disclosure provides nutritional compositions and methods for dietary management of the symptoms of celiac disease and/or food allergy via administration of a probiotic component comprising *Lactobacillus rhamnosus* GG (LGG). The LGG may be administered in a health-promoting nutritional composition that can alleviate the symptoms of celiac disease and/or food allergy by providing nutrients in a composition that contains few allergenic epitopes. The present disclosure further provides methods for providing nutritional support to subjects, such as infants, suffering from celiac disease and/or food allergy. A full and enabling disclosure of the claimed invention(s), including the best mode thereof directed to one of ordinary skill in the art, is set forth below.

The gut microflora in infants is known to be far less developed than that of an adult. While the microflora of the adult human consists of more than 10¹³ microorganisms and nearly 500 species, some being harmful and some being beneficial, the microflora of an infant contains only a fraction of those microorganisms, both in absolute number but also species diversity. Infants are born with a sterile gut, but infants acquire intestinal flora from the birth canal, from their environment, and from what they ingest. Because the gut microflora population is very unstable in early neonatal life, it is often difficult for the infant's gut to maintain the delicate balance between harmful and beneficial bacteria, thus reducing the ability of the immune system to function normally.

It is especially difficult for formula-fed infants to maintain this balance due to the differences between the bacterial species in the gut of a formula-fed and breast-fed infant. The stool of breast-fed infants contains predominantly *Bifidobacterium,* with *Streptococcus* and *Lactobacillus* as less common contributors. In contrast, the microflora of formula-fed infants is more diverse than that of breast fed infants containing *Bifidobacterium* and *Bacteroides* as well as the more pathogenic species, *Staphylococcus, Escherichia coli,* and *Clostridia.* The varied species of *Bifidobacterium* in the stools of breast-fed and formula-fed infants differ as well. A variety of factors have been proposed as the cause for the different fecal flora of breast-fed and formula-fed infants, including the lower content and different composition of proteins in human milk, a lower phosphorus content in human milk, the large variety of oligosaccharides in human milk, and numerous humoral and cellular mediators of immunologic function in breast milk. (Agostoni, et al., Probiotic Bacteria in Dietetic Products for Infants. A Commentary by the ESPGHAN Committee on Nutrition, J. Pediatr. Gastro. Nutr. 38:365-374 (April 2004)).

Because the microflora of formula-fed infants is so unstable and the gut microflora largely participates in stimulation of gut immunity, formula-fed infants are more likely to develop illnesses. Accordingly, studies have shown that celiac disease may appear earlier in formula-fed infants than in breastfed infants. Celiac disease causes damage to the small intestines and resulting nutrient absorption problems that only further compound health problems for formula-fed infants.

Furthermore, the diversity of total fecal microbiota as well as the species composition of lactic acid bacteria and *Bifidobacterium* have been shown to differ significantly between celiac children and age-matched controls. (Sanz et al. FEMS Immunol. Med. Micobiol. 2007 Dec; 51 (3):562-8). Indeed, studies have shown that the proportions of total bacteria and Gram-negative bacteria are significantly higher in celiac disease patients with active disease. (Nadal et al. J Med Microbiol. 2007 Dec; 56(Pt 12):1669-74). Overall, the higher incidence of Gram-negative and potentially pro-inflammatory bacteria in the duodenal microbiota of celiac children has been linked to symptomatic presentation of the disease and could favor the pathological process of the disorder. (Nadal et al. J Med Microbiol. 2007 Dec; 56(Pt 12):1669-74).

However, certain probiotic strains may exert protective effects against gluten/gliadins and therefore against the symptoms of celiac disease in infants and children. Indeed, probiotic bacteria may conserve the appearance of the tight junction, induce mucin secretion in epithelial cells or counteract the gliadin-induced formation of large membrane ruffles.

Different probiotic bacterial strains have their characteristic sets of peptidases, which may diverge from each other considerably and have variable substrate specificities. (Lindfors et al. Clin Exp Immunol. 2008 Jun; 152(3):552-558). For example, the peptidase repertoire of one strain could simply be more efficient than that of another in breaking up gliadin into small harmless peptide products. *Id.* However, the mechanisms regulating epithelial responses to probiotics are complex and mostly unknown.

Direct evidence of the role of specific *Lactobacillus* species in human disease has not previously been provided. (Sanz et al. FEMS Immunol. Med. Micobiol. 2007 Dec; 51(3):562-8). Yet, the inventors of the present disclosure have discovered that, when enterally administered, the specific strain *Lactobacillus rhamnosus* GG (LGG) can alleviate the symptoms of celiac disease and promote intestinal health. In some embodiments, LGG may promote mucin secretion by epithelial cells. In other embodiments, LGG may preserve the formation of the tight junction. In still other embodiments, LGG may counteract the formation of large membrane ruffles. Indeed, the present disclosure is directed to methods and nutritional compositions involving administration of an effective amount of LGG to alleviate the symptoms of celiac disease and/or food allergy and to promote healthy intestinal development and growth.

LGG is a probiotic strain isolated from healthy human intestinal flora. It was disclosed in U.S. Pat. No. 5,032,399 to Gorbach, et al., which is herein incorporated in its entirety, by reference thereto. LGG is resistant to most antibiotics, stable in the presence of acid and bile, and attaches avidly to mucosal cells of the human intestinal tract. It survives for 1-3 days in most individuals and up to 7 days in 30% of subjects. In addition to its colonization ability, LGG also beneficially affects mucosal immune responses. LGG is deposited with the depository authority American Type Culture Collection under accession number ATCC 53103.

The amount of LGG in the to be administered in a subject is an amount sufficient to provide or deliver the desired probiotic effect(s) that will ameliorate the symptoms of celiac disease and/or food allergy, as discussed above. A sufficient amount of LGG may vary within a broad range, depending on, for example, the total amount of cells of the LGG, the total daily dose desired and on other properties and ingredients of any embodiment of the nutritional composition(s) of the present disclosure. For example, some embodiments of the nutritional composition may comprise between about 10⁴ to 10¹² colony forming units (cfu) of LGG per gram formulation. In another embodiment, a daily dose of the nutritional composition may comprise about 10⁷ to about 10¹⁰ cfu of LGG per gram composition. In yet another embodiment, a daily dose of the nutritional composition of the present disclosure can comprise about 10⁸ to about 10¹¹ cfu of LGG per gram composition. In a particular embodiment, a daily dose of the nutritional composition of the present disclosure can comprise about 10⁹ cfu of LGG per gram composition.

Further, in some embodiments of the methods and compositions of the disclosure, an effective amount of LGG may correspond to between about 1×10⁴ and 1×10¹² cfu/L/kg/day. In another embodiment, an effective amount of LGG comprises the administration of between about 1×10⁸ and 1×10⁹ cfu/L/kg/day LGG. In yet another embodiment, the nutritional composition comprises the administration of about 1×10⁸ cfu/L/kg/day LGG.

The form of administration of LGG in the method and compositions of the disclosure is not critical, as long as a therapeutically effective amount is administered. Indeed, in some embodiments, the LGG can be encapsulated in a sugar, fat, or polysaccharide matrix to further increase the probability of bacterial survival. Compositions of the present disclosure can also be provided in a form suitable for consumption selected from the group consisting of beverage, milk, yogurt, fruit juice, fruit-based drink, chewable tablet, cookie, cracker, or a combination thereof.

If, during the first year of life, the subject is breast-fed, the administration of LGG can be via the mother's breast milk. In this embodiment, the mother can undergo LGG supplementation during the time period that she breast-feeds, thereby transferring an effective amount of LGG to her baby via breast milk.

If, during the first year of life, the subject is formula-fed, or if a mother supplements breast-feeding with formula-feeding, LGG can be supplemented into an infant formula which is then fed to the subject.

In an embodiment, the administration of LGG continues for at least 3 months. In another embodiment, the administration of LGG continues for at least 6 months. In yet another embodiment, the administration of LGG continues for at least 12 months. In a particular embodiment, the postnatal administration of LGG continues indefinitely.

In certain embodiments of the disclosure, the nutritional composition may contain one or more additional probiotics together with the LGG. Any probiotic known in the art may be acceptable in this embodiment provided it achieves the intended result. In a particular embodiment, the probiotic may be selected from any *Lactobacillus* species or any *Bifidobacterium* species, such as *Bifidobacterium longum,* and *Bifidobacterium animalis subsp. lactis* BB-12 (DSM No. 10140) or combinations thereof.

In an embodiment, the probiotic(s) of the present disclosure may be viable or non-viable. As used herein, the term "viable", refers to live microorganisms. The term "non-viable" or "non-viable probiotic" means non-living probiotic microorganisms, their cellular components and/or metabolites thereof. Such non-viable probiotics may have been heat-killed or otherwise inactivated, but they retain the ability to favorably influence the health of the host. The probiotics useful in the present disclosure may be naturally-occurring, synthetic or developed through the genetic manipulation of organisms, whether such new source is now known or later developed.

In some embodiments, inactivated LGG is utilized in the nutritional composition of the present disclosure. Inactivation may occur through any method currently known in the art. The inactivation may be accomplished, for example, via heat treatment, lyophilization, ultraviolet light, gamma radiation, pressure, chemical disruption, or mechanical disruption.

The form of administration of LGG, active and/or inactive, in the method and compositions of the present disclosure is not critical, as long an effective amount is administered. In some embodiments, the LGG is administered to a subject via a nutritional composition. In some embodiments, the LGG, active and/or inactive is administered to a subject in the form of tablets, pills, encapsulations, caplets, gel caps, capsules, oil drops, or sachets.

In an embodiment, inactivated LGG may be combined with one or more additional viable and/or inactivated probiotics to alleviate the symptoms of celiac disease and/or food allergy in a subject, such as in a formula-fed infant. Any live or inactivated probiotic known in the art may be acceptable in this embodiment provided it achieves the intended result. In a particular embodiment, the viable and/or inactivated probiotic is chosen from the group consisting of *Lactobacillus* and *Bifidobacterium.*

If a live probiotic is administered in combination with the inactivated probiotic, the amount of live probiotic may correspond to between about 1×10⁴ and 1×10¹² colony forming units (cfu) per kg body weight per day. In another embodiment, the live probiotics may comprise between about 1×10⁶ and 1×10⁹ cfu per kg body weight per day. In yet another embodiment, the live probiotics may comprise about 1×10⁸ cfu per kg body weight per day.

In addition, in some embodiments, live and inactivated LGG are administered in combination with one another. The combination of live and inactivated LGG is believed to provide complimentary or synergistic effects with regards to the anti-inflammatory properties of formulations containing these agents. While not wishing to be tied to this or any other theory, live probiotics such as LGG are thought to impart anti-inflammatory effects in part through interaction with specific receptors, known as Toll-like receptors (TLRs) on the surface of specific immune cells. Direct or indirect interaction between live LGG and these receptors initiates an intracellular signal transduction cascade that results in the alteration of gene expression in these target cells. It is this specific interaction and resulting alteration in gene expression and other cellular effects that is thought to be involved in the modulation of inflammation. Thus, because live and inactivated LGG are believed to operate through different mechanisms, it is believed that the combination of these components provides complimentary or synergistic anti-inflammatory effects.

Furthermore, the present disclosure provides methods and nutritional compositions that are useful to promote healthy intestinal growth and development of a subject. Indeed, in addition to a probiotic component comprising LGG, the nutritional composition(s) of the present disclosure may provide effective amounts of nutrients that will promote healthy development of a subject, such as a child or infant.

Specifically, the nutritional composition of the present disclosure may provide a subject with beneficial nutrients that are otherwise absent due to a variety of conditions, such as malnutrition resulting from the effects of celiac disease. Such nutrients include but are not limited to a lipid component, a protein component, a carbohydrate component and/or a prebiotic component.

Moreover, the nutritional composition may be administered one to two times daily or more frequently as directed by a medical professional. Administration may begin immediately after birth and may continue as long as a subject is in nutritional need.

The nutritional composition of the present disclosure may comprise a nutritional supplement, an infant formula, a children's formula, a growing up milk or any other ingestible nutritional product. Moreover, the disclosed nutritional composition may be provided in any form known in the art, such as a powder, a gel, a suspension, a paste, a solid, a liquid, a liquid concentrate, reconstituteable powdered milk substitute, or a ready-to-use product. In some embodiments, the nutritional composition is a liquid nutritional supplement suitable for enteral administration. In other embodiments, the nutritional composition is a powdered formula that is reconstitutable with water. In still other embodiments, the nutritional composition is a liquid fortifier that may be easily mixed with human milk or infant formula.

The nutritional composition comprises a lipid component that contains a fat or a combination of fats in order to deliver a desired blend of fatty acids to a subject. Suitable fat or lipid sources for practicing the present disclosure may be comprise any lipid source known in the art, including but not limited to, animal sources, e.g., milk fat, butter, butter fat, egg yolk lipid; marine sources, such as fish oils, marine oils, single-cell oils; vegetable and plant oils, such as corn oil, canola oil, sunflower oil, soybean oil, palmolein, coconut oil, high oleic sunflower oil, evening primrose oil, rapeseed oil, olive oil, flaxseed (linseed) oil, cottonseed oil, high oleic safflower oil, palm stearin, soy lecithin, palm kernel oil, wheat germ oil, medium chain triglyceride oil.

Moreover, all or part of the lipid component may comprise a lipid emulsion. In certain embodiments, the lipid component may comprise between about 2.5 and about 5 g per 100 mL of nutritional composition. In some embodiments the nutritional composition may comprise between about 5 and about 20% w/w of the lipid component.

In some embodiments, the nutritional composition includes a fatty acid component that comprises at least one fatty acid, such as a long-chain polyunsaturated fatty acid ("LCPUFA") or a combination of LCPUFAs. LCPUFAs generally have a carbon chain length of at least 18. Suitable LCPUFAs for inclusion in the nutritional composition include, but are not limited to, arachidonic acid ("ARA") and docosahexaenoic acid ("DHA"). In one embodiment, the lipid component comprises DHA. In another embodiment, the lipid component comprises ARA. In still other embodiments, the lipid component of the nutritional composition comprises both DHA and ARA. The preferred forms of DHA and ARA incorporated in the nutritional composition are free, non-esterified DHA and ARA.

The nutritional composition may comprise between about 0.3% and about 5% w/w DHA in some embodiments. In some embodiments, the nutritional composition includes an amount of DHA sufficient to deliver from about 3 mg per kg of body weight per day to about 150 mg per kg of body weight per day of DHA to a subject. In one embodiment, the amount is from about 6 mg per kg of body weight per day to about 100 mg per kg of body weight per day. In another embodiment the amount is from about 10 mg per kg of body weight per day to about 60 mg per kg of body weight per day. In yet another embodiment the amount is from about 15 mg per kg of body weight per day to about 30 mg per kg of body weight per day.

The source of DHA may be any source known in the art, such as, for example, marine oil, fish oil, single cell oil, egg yolk lipid, and brain lipid. The DHA can be in natural or refined form. Further, in one embodiment, the nutritional composition comprises a source of DHA comprising DHASCO® and/or a fungal oil blend.

Likewise, in some embodiments, the nutritional composition may be formulated to deliver at least about 25 mg/kg/day of docosahexaenoic acid to the subject. In some embodiments, the nutritional composition may be formulated to deliver at least about 50 mg/kg/day DHA. In other embodiments, the nutritional composition may deliver at least about 60 mg/kg/day of DHA to the subject. And in some embodiments, the nutritional composition may be formulated to deliver at least about 75 mg/kg/day of docosahexaenoic acid to the subject. In further embodiments, the nutritional composition is formulated to deliver at least about 100 mg/kg/day DHA. In some embodiments, the amount of DHA for use in the nutritional composition varies from about 5 mg/100 kcal to about 80 mg/100 kcal. In one embodiment, it varies from about 10 mg/100 kcal to about 50 mg/100 kcal; and in another embodiment from about 15 mg/100 kcal to about 20 mg/100 kcal. In a particular embodiment of the present disclosure, the amount of DHA is about 17 mg/100 kcal. In some embodiments, the nutritional composition will comprise between about 12 and about 200 mg of DHA per 100 mL.

The nutritional composition may comprise between about 0.5% and about 5% w/w ARA. In some embodiments, the amount of ARA in the nutritional composition is from about 5 mg per kg of body weight per day to about 150 mg per kg of body weight per day. In one embodiment, the amount varies from about 10 mg per kg of body weight per day to about 120 mg per kg of body weight per day. In another embodiment, the amount varies from about 15 mg per kg of body weight per day to about 90 mg per kg of body weight per day. In yet another embodiment, the amount varies from about 20 mg per kg of body weight per day to about 60 mg per kg of body weight per day.

Moreover, in some embodiments, the nutritional composition may be formulated to deliver at least about 25 mg/kg/day of arachidonic acid to the subject. In some embodiments, the nutritional composition may be formulated to deliver at least about 40 mg/kg/day ARA. In other embodiments, the nutritional composition may deliver at least about 50 mg/kg/day of ARA to the subject. And in some embodiments, the nutritional composition may be formulated to deliver at least about 60 mg/kg/day of ARA to the subject.

In some embodiments, the amount of ARA for use in the nutritional composition varies from about 10 mg/100 kcal to about 100 mg/100 kcal. In one embodiment, the amount of ARA varies from about 15 mg/100 kcal to about 70 mg/100 kcal. In another embodiment the amount of ARA varies from about 20 mg/100 kcal to about 40 mg/100 kcal. In a particular embodiment of the present disclosure, the amount of ARA is about 34 mg/100 kcal.

The ARA source may be any source of ARA known in the art. In some embodiments, the nutritional composition comprises a source of ARA comprising ARASCO® and/or a fungal oil blend. In some embodiments, the ARA component of the nutritional supplement comprises about 30% of a fungal oil blend.

The nutritional composition may be supplemented with both DHA and ARA as part of the lipid component. In some embodiments, the DHA:ARA ratio is between about 1:6 and 6:1. In other embodiments, the DHA:ARA ratio is between about 1:2 and 2:1. In still further embodiments, the DHA:ARA ratio is about 1:1. In still other embodiments, the DHA:ARA ratio may be from about 3:1 to about 1:9.

The protein component may comprise, but is not limited to, milk protein powders, milk protein concentrates, milk protein isolates, nonfat milk solids, nonfat milk, nonfat dry milk, whey protein, whey protein isolates, whey protein concentrates, sweet whey, acid whey, casein, acid casein, caseinate (*e.g*. sodium caseinate, sodium calcium caseinate, calcium caseinate) and any combinations thereof.

In an embodiment, the protein component of the nutritional composition may comprise intact proteins. In other embodiments, the protein component is provided as a combination of both intact proteins and partially hydrolyzed proteins, with a degree of hydrolysis of between about 4% and 10%. In still other embodiments, the proteins are more completely hydrolyzed. In further embodiments, the protein component comprises partially hydrolyzed protein. In another embodiment, the protein component comprises extensively hydrolyzed protein. In still another embodiment, the protein component of the nutritional composition consists essentially of extensively hydrolyzed protein in order to minimize the occurrence of food allergy. In yet another embodiment, the protein source may be supplemented with glutamine-containing peptides.

Some people exhibit allergies or sensitivities to intact proteins, i.e. whole proteins, such as those in intact cow's milk protein or intact soy protein isolate-based formulas. Many of these people with protein allergies or sensitivities are able to tolerate hydrolyzed protein. Hydrolysate formulas (also referred to as semi-elemental formulas) contain protein that has been hydrolyzed or broken down into short peptide fragments and amino acids and as a result is more easily digested. In people with protein sensitivities or allergies, immune system associated allergies or sensitivities often result in cutaneous, respiratory or gastrointestinal symptoms such as vomiting and diarrhea. People who exhibit reactions to intact protein formulas often will not react to hydrolyzed protein formulas because their immune system does not recognize the hydrolyzed protein as the intact protein that causes their symptoms.

Moreover, a recent study demonstrates the reaction of some celiac disease patients to intact proteins, particularly bovine casein, via serum IgA activity. (Cabrera-Chavez et al., Nutrition. 2009 Jun;25(6):715-716). It has been suggested that epitopes from bovine casein could be responsible for triggering symptoms of celiac disease. *Id.*

Thus, some gliadins and bovine caseins may share epitopes recognized by anti-gliadin IgA antibodies. Accordingly, then, the nutritional composition of the present disclosure reduces the incidence of food allergy, such as, for example, protein allergies and, consequently, the immune reaction of CD patients to proteins such as bovine casein, by providing a protein component comprising hydrolyzed proteins. A hydrolyzed protein component contains fewer allergenic epitopes than an intact protein component.

The nutritional composition comprises either partially or extensively hydrolyzed protein, such as protein from cow's milk. The hydrolyzed proteins may be treated with enzymes to break down some or most of the proteins that cause adverse symptoms with the goal of reducing allergic reactions, intolerance, and sensitization. Moreover, the proteins may be hydrolyzed by any method known in the art.

Because some gliadins and bovine caseins may share epitopes that trigger an immune response in CD patients, the nutritional composition of the present disclosure alleviates, treats, or prevents the symptoms of CD by providing a hydrolyzed protein component that is less likely to trigger an immune response because it contains fewer allergenic epitopes due to the hydrolysis of the proteins.

Hydrolyzed proteins (protein hydrolysates) for use in the methods and compositions of the present disclosure are proteins that have been hydrolyzed and broken down into shorter peptide fragments and amino acids, wherein the resulting degree of hydrolysis is at least about 20%, preferably from about 20% to about 80%. The term "hydrolyzed" as used herein, means a protein hydrolysate having a minimum degree of hydrolysis of at least about 20%, with the preferred ranges being referenced above. In the broadest sense, a protein has been hydrolyzed when one or more amide bonds have been broken. Breaking of amide bonds may occur unintentionally or incidentally during manufacture, for example due to heating or shear, but for purposes of the methods and compositions of the present disclosure, the term "hydrolyzed protein" simply means a protein which has been processed or treated in a manner intended to break amide bonds. Intentional hydrolysis may be affected, for example, by treating an intact protein with enzymes or acids.

The terms "protein hydrolysates" or "hydrolyzed protein" are used interchangeably herein and refer to hydrolyzed proteins, wherein the degree of hydrolysis is at least about 20%, preferably from about 20% to about 80%, more preferably from about 30% to about 80%, even more preferably from about 40% to about 60%. The degree of hydrolysis is the extent to which peptide bonds are broken by a hydrolysis method. The degree of protein hydrolysis for purposes of characterizing the hydrolyzed protein component of the nutritional composition is easily determined by one of ordinary skill in the formulation arts by quantifying the amino nitrogen to total nitrogen ratio (AN/TN) of the protein component of the selected formulation. The amino nitrogen component is quantified by USP titration methods for determining amino nitrogen content, while the total nitrogen component is determined by the Tecator Kjeldahl method, all of which are well known methods to one of ordinary skill in the analytical chemistry art.

When a peptide bond in a protein is broken by enzymatic hydrolysis, one amino group is released for each peptide bond broken, causing an increase in the amino nitrogen. It should be noted that even non-hydrolyzed protein would contain some exposed amino groups, and have an AN/TN ratio of greater than zero percent. Hydrolyzed proteins will also have a different molecular weight distribution than the non-hydrolyzed proteins from which they were formed. The functional and nutritional properties of hydrolyzed proteins can be affected by the different size peptides. A molecular weight profile is usually given by listing the percent by weight of particular ranges of molecular weight (in Daltons) fractions (e.g., 2,000 to 5,000 Daltons, greater than 5,000 Daltons). As previously mentioned, persons who exhibit sensitivity to whole or intact proteins can benefit from consumption of nutritional formulas containing hydrolyzed proteins. Such sensitive persons may especially benefit from the consumption of a hypoallergenic formula.

In some embodiments, the nutritional composition of the present disclosure is substantially free of intact proteins. In this context, the term "substantially free" means that the preferred embodiments herein comprise sufficiently low concentrations of intact protein to thus render the formula hypoallergenic. The extent to which a nutritional composition in accordance with the disclosure is substantially free of intact proteins, and therefore hypoallergenic, is determined by the August 2000 Policy Statement of the American Academy of Pediatrics in which a hypoallergenic formula is defined as one which in appropriate clinical studies demonstrates that it does not provoke reactions in 90% of infants or children with confirmed cow's milk allergy with 95% confidence when given in prospective randomized, double-blind, placebo-controlled trials.

Another alternative for subjects, such as infants, that have food allergy and/or milk protein allergies is a protein-free nutritional composition based upon amino acids. Amino acids are the basic structural building units of protein. Breaking the proteins down to their basic chemical structure by completely pre-digesting the proteins makes amino acid-based formulas the most hypoallergenic formulas available.

In a particular embodiment, the nutritional composition is protein-free and contains free amino acids as a protein equivalent source. In this embodiment, the amino acids may comprise, but are not limited to, histidine, isoleucine, leucine, lysine, methionine, cysteine, phenylalanine, tyrosine, threonine, tryptophan, valine, alanine, arginine, asparagine, aspartic acid, glutamic acid, glutamine, glycine, proline, serine, carnitine, taurine and mixtures thereof. In some embodiments, the amino acids may be branched chain amino acids. In other embodiments, small amino acid peptides may be included as the protein component of the nutritional composition. The amount of free amino acids in the nutritional composition may vary from about 1 to about 5 g/100 kcal. In an embodiment, 100% of the free amino acids have a molecular weight of less than 500 Daltons. In this embodiment, the nutritional formulation may be hypoallergenic.

In some embodiments, the nutritional composition comprises a carbohydrate component. The carbohydrates utilized in the nutritional composition may be any digestible carbohydrates, such as lactose, dextrose, fructose, sucrose, maltose, maltodextrin, corn syrup solids, or mixtures thereof, depending on usage. In some embodiments, corn syrup solids are preferred. Moreover, hydrolyzed, partially hydrolyzed, and/or extensively hydrolyzed carbohydrates may be desirable for inclusion in the nutritional composition due to their easy digestibility and to their ability to mitigate the effects of CD. Specifically, hydrolyzed carbohydrates are less likely to contain allergenic epitopes that trigger or worsen the symptoms of CD.

Non-limiting examples of carbohydrate materials suitable for use herein include hydrolyzed or intact, naturally or chemically modified, starches sourced from corn, tapioca, rice or potato, in waxy or non-waxy forms. Non-limiting examples of suitable carbohydrates include various hydrolyzed starches characterized as hydrolyzed cornstarch, maltodextrin, maltose, corn syrup, dextrose, corn syrup solids, glucose, and various other glucose polymers and combinations thereof. Non-limiting examples of other suitable carbohydrates include those often referred to as sucrose, lactose, fructose, high fructose corn syrup, indigestible oligosaccharides such as fructooligosaccharides and combinations thereof.

In one particular embodiment, the carbohydrate component of the nutritional composition is comprised of 100% lactose. In another embodiment, the carbohydrate component comprises between about 0% and 60% lactose. In another embodiment, the carbohydrate component is lactose-free, wherein it contains 0% lactose. In some embodiments, the carbohydrate component is galactose-free and/or sucrose-free, wherein it contains 0% galactose and/or sucrose, respectively. In another embodiment, the carbohydrate component comprises between about 15% and 55% lactose. In yet another embodiment, the carbohydrate component comprises between about 20% and 30% lactose. In these embodiments, the remaining source of carbohydrates may be any carbohydrate known in the art. In an embodiment, the carbohydrate component comprises about 25% lactose and about 75% corn syrup solids.

The nutritional composition may include a vitamin and mineral component. The vitamin and mineral component may optionally include, but is not limited to, one or more of the following minerals or derivations thereof: boron, calcium, calcium acetate, calcium gluconate, calcium chloride, calcium lactate, calcium phosphate, calcium sulfate, chloride, chromium, chromium chloride, chromium picolonate, copper, copper sulfate, copper gluconate, cupric sulfate, fluoride, iron, carbonyl iron, ferric iron, ferrous fumarate, ferric orthophosphate, iron trituration, polysaccharide iron, iodide, iodine, magnesium, magnesium carbonate, magnesium hydroxide, magnesium oxide, magnesium stearate, magnesium sulfate, manganese, molybdenum, phosphorus, potassium, potassium phosphate, potassium iodide, potassium chloride, potassium acetate, selenium, sulfur, sodium, docusate sodium, sodium chloride, sodium selenate, sodium molybdate, zinc, zinc oxide, zinc sulfate and mixtures thereof. Non-limiting exemplary derivatives of mineral compounds include salts, alkaline salts, esters and chelates of any mineral compound.

The minerals can be added to the nutritional product in the form of salts such as calcium phosphate, calcium glycerol phosphate, sodium citrate, potassium chloride, potassium phosphate, magnesium phosphate, ferrous sulfate, zinc sulfate, cupric sulfate, manganese sulfate, and sodium selenite. Additional vitamins and minerals can be added as known within the art.

The vitamin component of the nutritional composition may optionally include, but is not limited to, one or more of the following vitamins or derivations thereof: vitamin B₁ (thiamin, thiamin pyrophosphate, TPP, thiamin triphosphate, TTP, thiamin hydrochloride, thiamin mononitrate), vitamin B₂ (riboflavin, flavin mononucleotide, FMN, flavin adenine dinucleotide, FAD, lactoflavin, ovoflavin), vitamin B₃ (niacin, nicotinic acid, nicotinamide, niacinamide, nicotinamide adenine dinucleotide, NAD, nicotinic acid mononucleotide, NicMN, pyridine-3-carboxylic acid), vitamin B₃-precursor tryptophan, vitamin B₆ (pyridoxine, pyridoxal, pyridoxamine, pyridoxine hydrochloride), pantothenic acid (pantothenate, panthenol), folate (folic acid, folacin, pteroylglutamic acid), vitamin B₁₂ (cobalamin, methylcobalamin, deoxyadenosylcobalamin, cyanocobalamin, hydroxycobalamin, adenosylcobalamin), biotin, vitamin C (ascorbic acid), vitamin A (retinol, retinyl acetate, retinyl palmitate, retinyl esters with other long-chain fatty acids, retinal, retinoic acid, retinol esters), vitamin D (calciferol, cholecalciferol, vitamin D₃, 1,25,-dihydroxyvitamin D), vitamin E (α-tocopherol, α-tocopherol acetate, α-tocopherol succinate, α-tocopherol nicotinate, γ-tocopherol), vitamin K (vitamin K₁, phylloquinone, naphthoquinone, vitamin K₂, menaquinone-7, vitamin K₃, menaquinone-4, menadione, menaquinone-8, menaquinone-8H, menaquinone-9, menaquinone-9H, menaquinone-10, menaquinone-11, menaquinone-12, menaquinone-13), choline, inositol, β-carotene and any combinations thereof.

In certain embodiments, the nutritional composition of the present disclosure may comprise at least one prebiotic. In this embodiment, any prebiotic known in the art may be included. In a particular embodiment, the prebiotic can be selected from the group consisting of fructooligosaccharide, gluco-oligosaccharide, galactooligosaccharide, isomalto-oligosaccharide, xylo-oligosaccharide, lactulose, polydextrose and any combination thereof.

In some embodiments, polydextrose ("PDX") may be included in the nutritional composition in an amount sufficient to provide between about 1.0 g/L and 10.0 g/L. In another embodiment, the nutritional composition contains an amount of PDX that is between about 2.0 g/L and 8.0 g/L.

In a particular embodiment of the present disclosure, PDX is administered in combination with galactooligosaccharide ("GOS"). In this embodiment, PDX and GOS can be administered in a ratio of PDX:GOS of between about 9:1 and 1:9. In another embodiment, the ratio of PDX:GOS can be between about 5:1 and 1:5. In yet another embodiment, the ratio of PDX:GOS can be between about 1:3 and 3:1. In a particular embodiment, the ratio of PDX to GOS can be about 5:5. In another particular embodiment, the ratio of PDX to GOS can be about 8:2.

The amount of the PDX:GOS combination in the nutritional composition may be between about 1.0 g/L and 10.0 g/L. In another embodiment, the amount of the PDX:GOS combination may be between about 2.0 g/L and 8.0 g/L. In a particular embodiment, the amount of the PDX:GOS combination may be about 2 g/L of PDX and 2 g/L of GOS.

The nutritional composition of the present disclosure may also comprise an emulsifier. The emulsifier may comprise microencapsulants, surfactants, emulsion stabilizers or a combination thereof. In some embodiments, the emulsifier may comprise, for example, lecithin, mono-glyceride(s) or di-glyceride(s). In some embodiments, the lipid component of the nutritional composition provides fatty acid(s) in the form of a stable emulsion. In other embodiments the nutritional composition may comprise a stabilizer, such as carrageenan, in place of or in addition to an emulsifier.

Furthermore, some embodiments of the nutritional composition may mimic certain characteristics of human breast milk. However, to fulfill the specific nutrient requirements of some subjects, the nutritional composition may comprise a higher amount of some nutritional components than does human milk. For example, the nutritional composition may comprise a greater amount of DHA than does human breast milk. The enhanced level of DHA of the nutritional composition compensates for any existing nutritional DHA deficit.

Moreover, in some embodiments, the nutritional composition is nutritionally complete, containing suitable types and amounts of lipids, carbohydrates, proteins, vitamins and minerals to be a subject's sole source of nutrition. Indeed, the nutritional composition may optionally include any number of proteins, peptides, amino acids, fatty acids, probiotics and/or their metabolic by-products, prebiotics, carbohydrates and any other nutrient or other compound that may provide many nutritional and physiological benefits to a subject. Further, the nutritional composition of the present disclosure may comprise flavors, flavor enhancers, sweeteners, pigments, vitamins, minerals, therapeutic ingredients, functional food ingredients, food ingredients, processing ingredients or combinations thereof.

The present disclosure further provides a method for providing nutritional support to a subject. The method includes administering to the subject an effective amount of the nutritional composition of the present disclosure.

The nutritional composition may be expelled directly into a subject's intestinal tract. In some embodiments, the nutritional composition is expelled directly into the gut. In some embodiments, the composition may be formulated to be consumed or administered enterally under the supervision of a physician and may be intended for the specific dietary management of a disease or condition, such as celiac disease and/or food allergy, for which distinctive nutritional requirements, based on recognized scientific principles, are established by medical evaluation.

The nutritional composition of the present disclosure is not limited to compositions comprising nutrients specifically listed herein. Any nutrients may be delivered as part of the composition for the purpose of meeting nutritional needs and/or in order to optimize the nutritional status in a subject.

In some embodiments, the nutritional composition may be delivered to an infant from birth until at a time that matches full-term gestation. In some embodiments, the nutritional composition may be delivered to an infant until at least about three months corrected age. In another embodiment, the nutritional composition may be delivered to a subject as long as is necessary to correct nutritional deficiencies. In yet another embodiment, the nutritional composition may be delivered to an infant from birth until at least about six months corrected age. In yet another embodiment, the nutritional composition may be delivered to an infant from birth until at least about one year corrected age.

The nutritional composition of the present disclosure may be standardized to a specific caloric content, it may be provided as a ready-to-use product, or it may be provided in a concentrated form.

### EXAMPLES

The following examples are provided to illustrate embodiments of the nutritional composition of the present disclosure but should not be interpreted as a limitation thereon. The examples illustrate LGG-containing powdered infant formulas. In the examples, three major ingredients in infant formula are intermixed: powder base, corn syrup solids and protein hydrolysate. The component ingredients of the powder base for one embodiment of the infant formula are listed in Table 1.

**TABLE 1**

| Ingredient, unit | Per 100 kg base |
|---|---|
| Corn Syrup Solids, kg | 43.135 |
| Palm Olein Oil, kg | 16.2 |
| Modified Corn Starch, kg | 16.143 |
| Coconut Oil, kg | 7.2 |
| Soy Oil, kg | 7.2 |
| High Oleic Sunflower Oil, kg | 5.4 |
| Calcium Phosphate Dibasic, kg | 2.286 |
| Potassium Citrate, kg | 0.87 |
| Potassium Chloride, kg | 0.66 |
| Calcium Citrate, kg | 0.614 |
| Choline Chloride, kg | 0.154 |
| Magnesium Oxide Light, kg | 0.118 |
| L-Carnitine, g | 19.8 |
| Sodium Iodide, g | 0.119 |

An initial amount of LGG was added to the powder base of Table 1, corn syrup solids and protein hydrolysate mixture in order to prepare a product containing 6.25 x 10⁸ cfu/g product.

Table 2 shows an alternate embodiment of the powder base. An initial amount of LGG was added to the powder base of Table 2 in order to prepare a product containing 5.7 x 10⁷ cfu/g product.

**TABLE 2**

| Ingredients | Per 100 Calories (5 fl oz) |
|---|---|
| Protein, g | 2.8 |
| Fat, g | 5.3 |
| Carbohydrate, g | 10.3 |
| Water, g | 133 |
| Vitamin A, IU | 300 |
| Vitamin D, IU | 50 |
| Vitamin E, IU | 2 |
| Vitamin K, µg | 8 |
| Thiamin (Vitamin B1), µg | 80 |
| Riboflavin (Vitamin B2), µg | 90 |
| Vitamin B6, µg | 60 |
| Vitamin B12, µg | 0.3 |
| Niacin, µg | 1000 |
| Folic acid (folacin), µg | 16 |
| Pantothenic acid, µg | 500 |
| Biotin, µg | 3 |
| Vitamin C (ascorbic acid), mg | 12 |
| Choline, mg | 12 |
| Inositol, mg | 17 |
| Carnitine, mg | 2 |
| Taurine, mg | 6 |
| Calcium, mg | 94 |
| Phosphorus, mg | 63 |
| Magnesium, mg | 11 |
| Iron, mg | 1.8 |
| Zinc, mg | 1 |
| Manganese, µg | 25 |
| Copper, µg | 75 |
| Iodine, µg | 15 |
| Selenium, µg | 2.8 |
| Sodium, mg | 47 |
| Potassium, mg | 110 |
| Chloride, mg | 86 |

All references cited in this specification, including without limitation, all papers, publications, patents, patent applications, presentations, texts, reports, manuscripts, brochures, books, internet postings, journal articles, periodicals, and the like, are hereby incorporated by reference into this specification in their entireties. The discussion of the references herein is intended merely to summarize the assertions made by their authors and no admission is made that any reference constitutes prior art. Applicants reserve the right to challenge the accuracy and pertinence of the cited references.

## Claims

1. A nutritional composition comprising a protein component comprising extensively hydrolyzed protein and at least one probiotic, wherein the at least one probiotic comprises *Lactobacillus rhamnosus* GG, for use in a method of promoting healthy intestinal development in an infant suffering from celiac disease.

2. The nutritional composition for use according to claim 1, wherein the nutritional composition further comprises at least one long chain polyunsaturated fatty acid.

3. The nutritional composition for use according to claim 2, wherein the at least one long chain polyunsaturated fatty acid comprises docosahexaenoic acid.

4. The nutritional composition for use according to claim 2, wherein the at least one long chain polyunsaturated fatty acid comprises docosahexaenoic acid and arachidonic acid.

5. The nutritional composition for use according to claim 1, wherein the nutritional composition further comprises at least one prebiotic.

6. The nutritional composition for use according to claim 1, wherein protein component consists essentially of extensively hydrolyzed protein.

7. A nutritional composition comprising a protein component comprising hydrolyzed protein and at least one probiotic for use in alleviating the symptoms of celiac disease in an infant suffering from celiac disease wherein the nutritional composition further comprises a lipid component and the at least one probiotic comprises *Lactobacillus rhamnosus* GG.

8. The nutritional composition for use of claim 7, wherein the nutritional composition further comprises at least one long chain polyunsaturated fatty acid.

9. The nutritional composition for use of claim 7, wherein the nutritional composition further comprises at least one prebiotic.

10. The nutritional composition for use of claim 7, wherein the protein component consists essentially of extensively hydrolyzed protein.

11. The nutritional composition for use of claim 7, wherein the at least one long chain polyunsaturated fatty acid comprises docosahexaenoic acid.

12. The nutritional composition for use of claim 7, wherein the hydrolyzed protein has a degree of hydrolysis of 20% to 80%.

13. The nutritional composition for use of claim 7, wherein the hydrolyzed protein has a degree of hydrolysis of 30% to 60%.

## Patentansprüche

1. Ernährungszusammensetzung, umfassend eine Proteinkomponente, die stark hydrolysiertes Protein umfasst, und wenigstens ein Probiotikum, wobei das wenigstens eine Probiotikum *Lactobacillus rhamnosus* GG umfasst, für die Verwendung bei einem Verfahren zum Fördern einer gesunden Darmentwicklung bei einem Säugling, der an Zöliakie leidet.

2. Ernährungszusammensetzung für die Verwendung gemäß Anspruch 1, wobei die Ernährungszusammensetzung ferner wenigstens eine langkettige, mehrfach ungesättigte Fettsäure umfasst.

3. Ernährungszusammensetzung für die Verwendung gemäß Anspruch 2, wobei die wenigstens eine langkettige, mehrfach ungesättigte Fettsäure Docosahexaensäure umfasst.

4. Ernährungszusammensetzung für die Verwendung gemäß Anspruch 2, wobei die wenigstens eine langkettige, mehrfach ungesättigte Fettsäure Docosahexaensäure und Arachidonsäure umfasst.

5. Ernährungszusammensetzung für die Verwendung gemäß Anspruch 1, wobei die Ernährungszusammensetzung ferner wenigstens ein Präbiotikum umfasst.

6. Ernährungszusammensetzung für die Verwendung gemäß Anspruch 1, wobei die Proteinkomponente im Wesentlichen aus stark hydrolysiertem Protein besteht.

7. Ernährungszusammensetzung, umfassend eine Proteinkomponente, die hydrolysiertes Protein umfasst, und wenigstens ein Probiotikum, für die Verwendung zum Mildern der Symptome von Zöliakie bei einem Säugling, der an Zöliakie leidet, wobei die Ernährungszusammensetzung ferner eine Lipidkomponente umfasst und das wenigstens eine Probiotikum *Lactobacillus rhamnosus* GG umfasst.

8. Ernährungszusammensetzung für die Verwendung gemäß Anspruch 7, wobei die Ernährungszusammensetzung ferner wenigstens eine langkettige, mehrfach ungesättigte Fettsäure umfasst.

9. Ernährungszusammensetzung für die Verwendung gemäß Anspruch 7, wobei die Ernährungszusammensetzung ferner wenigstens ein Präbiotikum umfasst.

10. Ernährungszusammensetzung für die Verwendung gemäß Anspruch 7, wobei die Proteinkomponente im Wesentlichen aus stark hydrolysiertem Protein besteht.

11. Ernährungszusammensetzung für die Verwendung gemäß Anspruch 7, wobei die wenigstens eine langkettige, mehrfach ungesättigte Fettsäure Docosahexaensäure umfasst.

12. Ernährungszusammensetzung für die Verwendung gemäß Anspruch 7, wobei das hydrolysierte Protein einen Hydrolysegrad von 20 % bis 80 % aufweist.

13. Ernährungszusammensetzung für die Verwendung gemäß Anspruch 7, wobei das hydrolysierte Protein einen Hydrolysegrad von 30 % bis 60 % aufweist.

## Revendications

1. Composition nutritionnelle comprenant un composant protéique comprenant des protéines fortement hydrolysées et au moins un probiotique, où le au moins un probiotique comprend *Lactobacillus rhamnosus GG,* pour une utilisation dans une méthode destinée à favoriser un développement intestinal sain chez un nourrisson souffrant de la maladie coeliaque.

2. Composition nutritionnelle pour une utilisation selon la revendication 1, où la composition nutritionnelle comprend en outre au moins un acide gras polyinsaturé à chaîne longue.

3. Composition nutritionnelle pour une utilisation selon la revendication 2, où le au moins un acide gras polyinsaturé à chaîne longue comprend de l'acide docosahexaénoïque.

4. Composition nutritionnelle pour une utilisation selon la revendication 2, où le au moins un acide gras polyinsaturé à chaîne longue comprend de l'acide docosahexaénoïque et de l'acide arachidonique.

5. Composition nutritionnelle pour une utilisation selon la revendication 1, où la composition nutritionnelle comprend en outre au moins un prébiotique.

6. Composition nutritionnelle pour une utilisation selon la revendication 1, où le composant protéique est constitué essentiellement de protéines fortement hydrolysées.

7. Composition nutritionnelle comprenant un composant protéique comprenant des protéines hydrolysées et au moins un probiotique pour une utilisation dans le soulagement des symptômes de la maladie coeliaque chez un nourrisson souffrant de la maladie coeliaque, où la composition nutritionnelle comprend en outre un composant lipidique et le au moins un probiotique comprend *Lactobacillus rhamnosus GG.*

8. Composition nutritionnelle pour une utilisation selon la revendication 7, où la composition nutritionnelle comprend en outre au moins un acide gras polyinsaturé à chaîne longue.

9. Composition nutritionnelle pour une utilisation selon la revendication 7, où la composition nutritionnelle comprend en outre au moins un prébiotique.

10. Composition nutritionnelle pour une utilisation selon la revendication 7, où le composant protéique est constitué essentiellement de protéines fortement hydrolysées.

11. Composition nutritionnelle pour une utilisation selon la revendication 7, où le au moins un acide gras polyinsaturé à chaîne longue comprend de l'acide docosahexaénoïque.

12. Composition nutritionnelle pour une utilisation selon la revendication 7, où les protéines hydrolysées possèdent un degré d'hydrolyse allant de 20% à 80%.

13. Composition nutritionnelle pour une utilisation selon la revendication 7, où les protéines hydrolysées possèdent un degré d'hydrolyse allant de 30% à 60%.
